# EUROPEAN PATENT APPLICATION

(11) **EP 3 838 235 A1**
(43) Date of publication of application: **23.06.2021**
(21) Application number: 20210184.6
(22) Date of filing: 27.11.2020
(51) Int. Cl.: A61F 5/442, A61F 5/451

(54) **EXCREMENT CARE ROBOT WITH PRECISION CONTROL FUNCTION OF ADJACENT REGION TO THE GENITALS FOR MAKING OPTIMAL ENVIRONMENT INSIDEWEARING PART OF BODY-FITTED TYPE TREATING APPARATUS**

(30) Priority: 18.12.2019 KR 20190169835
(71) Applicant: Curaco, Inc., Gyeonggi-do, 13210 (KR)
(72) Inventor: LEE, Hoonsang, 12787 Gyeonggi-do (KR); LEE, Hosang, 06218 Seoul (KR); CHOI, Sungpil, 07257 Seoul (KR); LEE, Dounghoon, 12797 Gyeonggi-do (KR)
(74) Representative: Ter Meer Steinmeister & Partner

(57) **Abstract**

Disclosed is an excrement care robot with a precision control function of an adjacent region to the genitals for making an optimal wearing environment inside a wearing part of a body-fitted type treating apparatus, the excrement care robot including: a body-fitted type treating apparatus including a body which has a shape corresponding to bends of the genitals and buttocks of the human body and is formed with a treatment space that is opened in a direction of the genitals and buttocks of the human body to receive excrement discharged from the human body, a discharge unit which is provided in the body and communicates with the treatment space to discharge the excrement of the treatment space to the outside, a sensor unit which is provided in the body to sense an environment condition in the treatment space including a temperature and a humidity, and an environment control unit which is provided in the body to control the temperature and humidity environments of the treatment space; and a motion analysis unit that receives the sensor data of the sensor unit to analyze the motion of the human body through the motion of the body.

## Description

### BACKGROUND

### Technical Field

The present invention relates to an excrement care robot that is formed to be wearable on a human body to treat excrement, and more particularly, to an excrement care robot with a precision control function of an adjacent region to the genitals so as to make an environment inside a wearing part of a body-fitted type treating apparatus to an optimal wearing environment.

### Background Art

In general, people such as patients, the elderly, etc., who have difficulty in moving or who have difficulty moving their lower bodies at will, have no ability capable of treating excrement on their own, so that there is a discomfort that a guardian or caregiver must always reside around the people.

Therefore, in order to solve such discomfort, the research and development of an excrement treating apparatus that comes into direct contact with the human body to collect the excrement have been conducted. Such an excrement treating apparatus is designed to receive the user's excrement, suck the received excrement, and then discharge the sucked excrement to the outside, so that the excrement can be automatically treated even if a guardian or caregiver does not reside around the user.

However, conventional excrement treating apparatuses which have been developed up to date often focus on only an excrement treatment function, and are designed without considering the user's body, and thus, there is a problem that the usability is very poor. In general, the periphery of the buttocks or genitals from which the excrement is excreted has a structure that is difficult to be in close contact with the excrement treating apparatus due to severe bends, and accordingly, there are many cases where the excrement leaks between the human body and the excrement treating apparatus.

In addition, due to the features of the excrement treating apparatus, which is continuously worn, the environment around the buttocks and genitals is highly likely to be left in a humid and high-temperature condition, and when this condition is maintained, there is a problem that the condition adversely affects the patient's health.

Therefore, there is a need for a method for solving these problems.

[Prior Art Document] Korean Patent No. 10-0893733

The above information disclosed in this Background section is only for enhancement of understanding of the background of the invention and therefore it may contain information that does not form the prior art that is already known in this country to a person of ordinary skill in the art.

### SUMMARY OF THE DISCLOSURE

The present invention has been made in an effort to solve the above-described problems associated with prior art. An object of the present invention is to improve a user's wearing feeling and maintain health by making an environment inside a wearing part of a body-fitted type treating apparatus which is an adjacent space to the genitals to an optimal wearing environment.

The objects of the present invention are not limited to the aforementioned objects, and other objects, which are not mentioned above, will be apparently understood to those skilled in the art from the following description.

The object is solved by the features of the independent claims. Preferred embodiments are given in the dependent claims,

In order to achieve the objects described above, according to an embodiment of the present invention, there is provided an excrement care robot with a precision control function of an adjacent region to the genitals for making an optimal wearing environment inside a wearing part of a body-fitted type treating apparatus, the excrement care robot including: a body-fitted type treating apparatus including a body which has a shape corresponding to bends of the genitals and buttocks of the human body and is formed with a treatment space that is opened in a direction of the genitals and buttocks of the human body to receive excrement discharged from the human body, a discharge unit which is provided in the body and communicates with the treatment space to discharge the excrement of the treatment space to the outside, a sensor unit which is provided in the body to sense an environment condition in the treatment space including a temperature and a humidity, and an environment control unit which is provided in the body to control the temperature and humidity environments of the treatment space; and a driving control unit which receives sensor data of the sensor unit to control the driving of the environment control unit.

The sensor unit may include a temperature measuring sensor that measures the temperature in the treatment space, and a humidity measuring sensor that measures the humidity in the treatment space.

The environment control unit may include an air suck passage for sucking air in the treatment space, a spray unit for spraying hot or cold water into the treatment space, an air supply passage for supplying air having predetermined temperature and humidity conditions to the treatment space, and a heating heater for heating the air supplied through the air supply passage.

When the temperature of the treatment space is less than a preset reference temperature and the humidity of the treatment space is within a preset reference humidity range, the driving control unit may control the heating heater and the air supply passage to be driven at the same time.

When the temperature of the treatment space is less than the preset reference temperature and the humidity of the treatment space is less than the preset reference humidity range, the driving control unit may control the heating heater and the air supply passage to be driven at the same time and control the spray unit to spray the hot water.

When the temperature of the treatment space is less than the preset reference temperature and the humidity of the treatment space is more than the preset reference humidity range, the driving control unit may control the heating heater, the air supply passage, and the air suck passage to be driven at the same time.

When the temperature of the treatment space is equal to or more than the preset reference temperature and the humidity of the treatment space is within the preset reference humidity range, the driving control unit may control the air suck passage to be driven.

When the temperature of the treatment space is equal to or more than the preset reference temperature and the humidity of the treatment space is less than the preset reference humidity range, the driving control unit may control the air suck passage and the air supply passage to be driven at the same time and control the spray unit to spray the cold water.

When the temperature of the treatment space is equal to or more than the preset reference temperature and the humidity of the treatment space is more than the preset reference humidity range, the driving control unit may control the air suck passage and the air supply passage to be driven at the same time.

According to the present invention, the excrement care robot with a precision control function of an adjacent region to the genitals for making an optimal wearing environment inside a wearing part of a body-fitted type treating apparatus may make an optimal wearing environment by maintaining an environment of a treatment space provided inside the wearing part which is a space adjacent to the genitals at appropriate temperature and humidity, thereby improving a user's wearing feeling and preventing adverse effects on the skin from occurring due to contamination of a user's body by the waste.

The effects of the present invention are not limited to the aforementioned effect, and other effects not mentioned above will be clearly understood to those skilled in the art from the description of the appended claims.

### BRIEF DESCRIPTION OF THE DRAWINGS

The above and other features of the present invention will now be described in detail with reference to certain exemplary embodiments thereof illustrated the accompanying drawings which are given hereinbelow by way of illustration only, and thus are not limitative of the present invention, and wherein:
FIG. 1 is a view illustrating an appearance of a body-fitted type treating apparatus in an excrement care robot according to an embodiment of the present invention;
FIG. 2 is a view illustrating a configuration of an environment control unit provided in a treatment space of the body-fitted type treating apparatus in the excrement care robot according to an embodiment of the present invention;
FIG. 3 is a view illustrating all components of the excrement care robot according to an embodiment of the present invention;
FIG. 4 is a view illustrating an appearance of wearing the body-fitted type treating apparatus on a human body in the excrement care robot of an embodiment of the present invention; and
FIG. 5 is a view illustrating a table of summarizing an algorithm of a driving control unit in the excrement care robot according to an embodiment of the present invention.

It should be understood that the appended drawings are not necessarily to scale, presenting a somewhat simplified representation of various preferred features illustrative of the basic principles of the invention. The specific design features of the present invention as disclosed herein, including, for example, specific dimensions, orientations, locations, and shapes will be determined in part by the particular intended application and use environment.

In the figures, reference numbers refer to the same or equivalent parts of the present invention throughout the several figures of the drawing.

### DETAILED DESCRIPTION

Hereinafter, a preferred embodiment of the present invention, of which an object of the present invention may be realized in detail, will be described with reference to the accompanying drawings. In describing the embodiment, like names and like reference numerals will be used with respect to like components and the resulting additional description will be omitted.

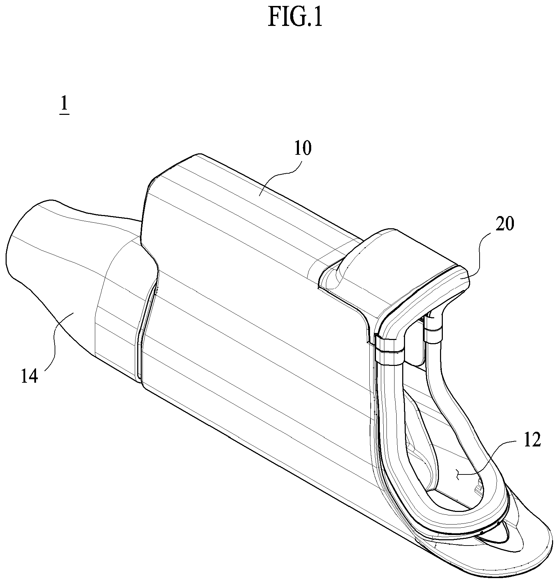

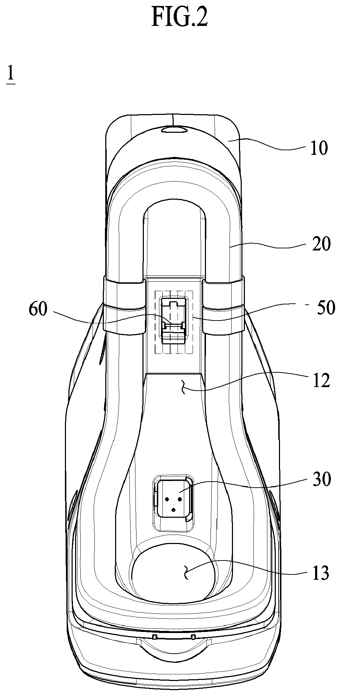

FIG. 1 is a view illustrating an appearance of an excrement care robot according to an embodiment of the present invention and FIG. 2 is a view illustrating a configuration of an environment control unit provided in a treatment space in the excrement care robot according to an embodiment of the present invention.

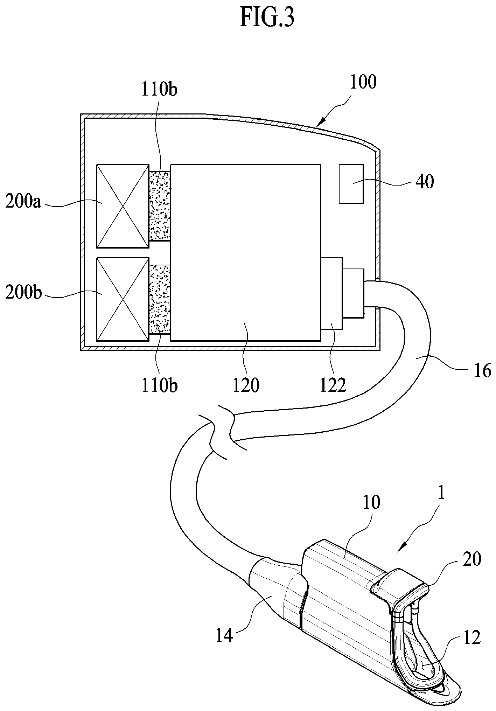

In addition, FIG. 3 is a view illustrating all components of the excrement care robot according to an embodiment of the present invention.

As illustrated in FIGS. 1 to 3, an excrement care robot according to an embodiment of the present invention includes a body-fitted type treating apparatus 1 that is a component directly worn by a user, that is, a bed-ridden patient, and a main treating device 100 connected with the body-fitted type treating apparatus.

In addition, the body-fitted type treating apparatus 1 includes a body 10, a discharge unit 14, and a contact member 20.

The body 10 has a shape corresponding to bends of the genitals and buttocks of the human body, and includes a wearing part formed with a treatment space 12 that is opened in a direction of the genitals and buttocks of the human body to receive excrement discharged from the human body, and a main body part which is connected to the wearing part so as to be fitted between the legs of the human body and has a receiving space formed therein.

Here, the genitals of the human body refer to an area around the genitals of men and women, and the buttocks refer to an area around the anus connected with the genitals. That is, the wearing part is formed to be fitted on the groin of the human body, and has a curved shape to correspond to the bends thereof.

In addition, in order to maximize the wearing feeling when the wearing part is fitted on the human body and to facilitate the measurement of vacuum pressure to be described below, the contact member 20 having elasticity may be provided in the wearing part.

In addition, although not illustrated in the drawing, a receiving space is provided inside the main body part, and various components required for the excrement treating apparatus may be provided in the receiving space. As such, the components provided in the receiving space are obvious to those skilled in the art, and since the structure thereof may be variously formed without limitation, a detailed description thereof will be omitted.

The discharge unit 14 is provided at the rear of the body 10 and communicates with the treatment space 12 through a discharge passage to discharge the excrement of the treatment space 12 to the outside.

In particular, in the embodiment, the discharge unit 14 protrudes to the rear side of the body 10 to be provided on the main treating device 100 side, and may be connected with a waste tank 120 having a collection space formed therein so that the excrement discharged by the discharge unit 14 is collected through an excrement flow hose 16.

In addition, the excrement is sucked into the waste tank 120 side by a suck force generated from blow motors 200a and 200b which are provided in the main treating device 100 to provide a vacuum suck force on the flow passage of the excrement.

Meanwhile, the embodiment further includes a sensor unit (not illustrated) which is provided in the body to sense environmental conditions in the treatment space including temperature and humidity, environment control units 13, 30, 50, and 60 which are provided in the body to control the temperature and humidity environments of the treatment space, and a driving control unit 40 which receives sensor data of the sensor unit to control the driving of the environment control units 13, 30, 50, and 60.

In the embodiment, the sensor unit may include a temperature measuring sensor that measures the temperature in the treatment space, and a humidity measuring sensor that measures the humidity in the treatment space.

Further, in the embodiment, the environment control units 13, 30, 50, and 60 may include an air suck passage 13 for sucking air in the treatment space 12, a spray unit 30 for spraying hot or cold water into the treatment space 12, an air supply passage 60 for supplying air having predetermined temperature and humidity conditions to the treatment space 12, and a heating heater 50 for heating the air supplied through the air supply passage 60.

More specifically, in the embodiment, the air suck passage 13 may have the same configuration as a discharge passage for discharging the excrement of the treatment space 12 to the outside.

In addition, a blower may be provided inside the air supply passage 60 to supply external air into the treatment space 12. In addition, the heating heater 50 is provided inside the air supply passage 60 to heat the air supplied through the air supply passage 60.

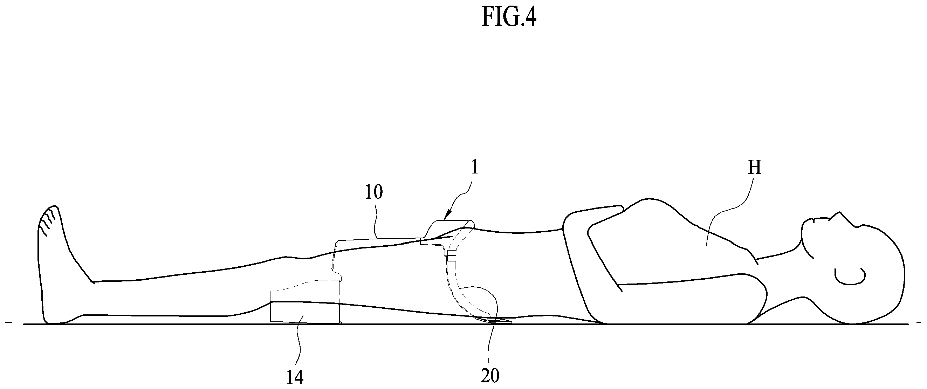

FIG. 4 is a view illustrating an appearance of wearing the body-fitted type treating apparatus 100 on a human body in the excrement care robot according to an embodiment of the present invention.

As illustrated in FIG. 4, a user H may stably wear the body-fitted type treating apparatus 1 of the excrement care robot by contacting a fitting part of the body 10 of the body-fitted type treating apparatus 1 with the genitals and buttocks in a state lying on a bed and extending user's legs to both sides of the main body part.

As such, since the body-fitted type treating apparatus 1 of the excrement care robot according to the embodiment is formed to correspond to the shape of the human body, the user H does not need to forcefully change a posture in accordance with the body-fitted type treating apparatus 1, but may maintain a natural posture.

In addition, the driving control unit 40 may be provided in various positions.

For example, the driving control unit 40 may be directly embedded in the body 10 of the body-fitted type treating apparatus 1, or also provided on the main treating device 100 side. Alternatively, the driving control unit 40 may also be provided in an external terminal or the like separately from the body-fitted type treating apparatus 1 and the main treating device 100.

In the case of the embodiment, the driving control unit 40 is provided on the main treating device 100 side.

Hereinafter, an algorithm of the driving control unit 40 that receives the sensor data of the sensor unit to control the driving of the environment control units 13, 30, 50, and 60 will be described in detail.

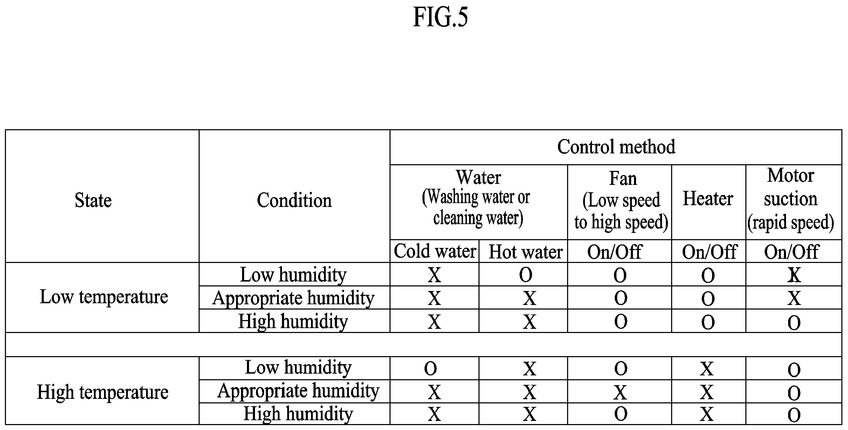

In addition, FIG. 5 is a view illustrating a table of summarizing an algorithm of the driving control unit.

As illustrated in FIG. 5, the driving control unit compares the temperature of the treatment space 12 with a preset reference temperature, and compares the humidity of the treatment space 12 with a preset reference humidity range to control the driving of the environment control units 13, 30, 50, and 60.

Specifically, when the temperature of the treatment space 12 is less than the preset reference temperature and the humidity of the treatment space 12 is within the preset reference humidity range, the driving control unit may control the heating heater 50 and the air supply passage 60 to be driven at the same time.

In this case, since the humidity of the treatment space 12 is maintained in an appropriate range, but the temperature is low, the driving control unit supplies external air having a higher temperature than the air in the treatment space 12 through the air supply passage 60 by driving the heating heater 50.

In addition, when the temperature of the treatment space 12 is less than the preset reference temperature and the humidity of the treatment space 12 is less than the preset reference humidity range, the driving control unit may control the heating heater 50 and the air supply passage 60 to be driven at the same time and control the spray unit 30 to spray the hot water.

In this case, since both the humidity and the temperature of the treatment space 12 are low, the driving control unit may supply the external air having a higher temperature than the air in the treatment space 12 through the air supply passage 60 by driving the heating heater 50 and increase the humidity of the treatment space 12 by spraying the hot water through the spray unit 30.

Further, when the temperature of the treatment space 12 is less than the preset reference temperature and the humidity of the treatment space 12 is more than the preset reference humidity range, the driving control unit may control the heating heater 50, the air supply passage 60, and the air suck passage 13 to be driven at the same time.

In this case, since the temperature of the treatment space 12 is low and the humidity thereof is excessively high, the driving control unit supplies the external air having a higher temperature than the air in the treatment space 12 through the air supply passage 60 by driving the heating heater 50 and sucks air having a high humidity in the treatment space 12 to discharge the air to the outside, thereby appropriately maintaining the humidity condition.

Next, when the temperature of the treatment space 12 is equal to or more than the preset reference temperature and the humidity of the treatment space 12 is within the preset reference humidity range, the driving control unit may control the air suck passage 13 to be driven.

In this case, when the humidity of the treatment space 12 is maintained in an appropriate range, but the temperature is high, the driving control unit may suck air having a high temperature in the treatment space 12 through the air suck passage 13 and discharge the sucked air to the outside to maintain the temperature condition appropriately.

In addition, when the temperature of the treatment space 12 is equal to or more than the preset reference temperature and the humidity of the treatment space 12 is less than the preset reference humidity range, the driving control unit may control the air suck passage 13 and the air supply passage 60 to be driven at the same time and control the spray unit 30 to spray the cold water.

In this case, since the humidity of the treatment space 12 is low, but the temperature thereof is high, the driving control unit supplies external air having a lower temperature than the air in the treatment space 12 through the air suck passage 13, sucks air having a high temperature in the treatment space 12 through the air suck passage 13 and discharge the sucked air to the outside to maintain the temperature condition appropriately, and simultaneously, sprays the cold water through the spray unit 30 to increase the humidity of the treatment space 12.

Further, when the temperature of the treatment space 12 is equal to or more than the preset reference temperature and the humidity of the treatment space 12 is more than the preset reference humidity range, the driving control unit may control the air suck passage 13 and the air supply passage 60 to be driven at the same time.

In this case, since both the humidity and the temperature of the treatment space 12 are high, the driving control unit supplies external air having lower temperature and humidity than the air in the treatment space 12 through the air suck passage 13 and sucks air having high temperature and humidity in the treatment space 12 through the air suck passage 13 and discharge the sucked air to the outside to maintain the temperature and humidity conditions appropriately.

Meanwhile, in the present invention, in addition to the above-listed methods, the environment control units 13, 30, 50, and 60 may be driven in various methods in various situations.

For example, when the temperature of the treatment space 12 is equal to or more than the preset reference temperature, the driving control unit may operate only the suck function through the air suck passage 13, supplies the external air through only the air supply passage 60 while the heating heater 50 is not driven, or operates only the spray unit 30 to spray the cold water, thereby lowering the temperature.

In particular, when the temperature of the treatment space 12 is excessively higher than the preset reference temperature, all of the listed components may be operated to quickly control the temperature.

As described above, since the optimal wearing environment may be made by maintaining the appropriate temperature and humidity in the treatment space 12 inside the wearing part of the body-fitted type treating apparatus 1 adjacent to the genitals, the present invention has advantages of improving a user's wearing feeling and preventing adverse effects on the skin from occurring due to contamination of the user's body by the waste.

As described above, the prepared embodiment of the present invention has been described, and in addition to the embodiments described above, a fact that the present invention can be materialized in other specific forms without departing from the gist or scope thereof will be apparent to those skilled in the art. Therefore, the aforementioned embodiments are not limited but should be considered to be illustrative, and accordingly, the present invention is not limited to the aforementioned description and will be modified within the scope of the appended claims and a range equivalent thereto.

## Claims

1. An excrement care robot with a precision control function of an adjacent region to the genitals for making an optimal wearing environment inside a wearing part of a body-fitted type treating apparatus (1), the excrement care robot comprising:
a body-fitted type treating apparatus (19 including a body (10) which has a shape corresponding to bends of the genitals and buttocks of the human body and having a treatment space (12) that is opened in a direction of the genitals and buttocks of the human body to receive excrement discharged from the human body,
a discharge unit (14) provided in the body (10) and configured to communicate with the treatment space (12) to discharge the excrement of the treatment space (12) to the outside,
a sensor unit provided in the body (10) to sense an environment condition in the treatment space (12) including a temperature and/or a humidity, and
an environment control unit (13, 30, 50, 60) provided in the body (10) to control the temperature and/or humidity environments of the treatment space (12); and
a driving control unit (40) configured to receive sensor data of the sensor unit to control the driving of the environment control unit (13, 30, 50, 60).

2. The excrement care robot of claim 1, wherein the sensor unit comprises at least one of:
a temperature measuring sensor configured to measure the temperature in the treatment space (12); and
a humidity measuring sensor configured to measure the humidity in the treatment space (12).

3. The excrement care robot of claim 1 or 2, wherein the environment control unit (13, 30, 50, 60) comprises at least one of:
an air suck passage (13) for sucking air in the treatment space (12);
a spray unit (30) for spraying hot or cold water into the treatment space (12);
an air supply passage (60) for supplying air having predetermined temperature and/or humidity conditions to the treatment space (12); and
a heater (50) for heating the air supplied through the air supply passage (13).

4. The excrement care robot of claim 3, wherein when the temperature of the treatment space (12) is less than a preset reference temperature and the humidity of the treatment space (12) is within a preset reference humidity range, the driving control unit (40) is configured to control the heater (50) and the air supply passage (13) to be driven at the same time.

5. The excrement care robot of claim 3, wherein when the temperature of the treatment space (12) is less than the preset reference temperature and the humidity of the treatment space (12) is less than the preset reference humidity range, the driving control unit (40) is configured to control the heater (50) and the air supply passage (60) to be driven at the same time and controls the spray unit (30) to spray hot water.

6. The excrement care robot of claim 3, wherein when the temperature of the treatment space (12) is less than the preset reference temperature and the humidity of the treatment space (12) is more than the preset reference humidity range, the driving control unit (40) is configured to control the heater (50), the air supply passage (60), and the air suck passage (13) to be driven at the same time.

7. The excrement care robot of claim 3, wherein when the temperature of the treatment space (12) is equal to or more than the preset reference temperature and the humidity of the treatment space (12) is within the preset reference humidity range, the driving control unit (40) is configured to control the air suck passage (13) to be driven.

8. The excrement care robot of claim 3, wherein when the temperature of the treatment space (12) is equal to or more than the preset reference temperature and the humidity of the treatment space (12) is less than the preset reference humidity range, the driving control unit (40) is configured to control the air suck passage (13) and the air supply passage (60) to be driven at the same time and to control the spray unit (30) to spray cold water.

9. The excrement care robot of claim 3, wherein when the temperature of the treatment space (12) is equal to or more than the preset reference temperature and the humidity of the treatment space (12) is more than the preset reference humidity range, the driving control unit (40) is configured to control the air suck passage (13) and the air supply passage (60) to be driven at the same time.
